# EUROPEAN PATENT APPLICATION

(11) **EP 1 366 784 A1**
(43) Date of publication of application: **03.12.2003**
(21) Application number: 03253296.2
(22) Date of filing: 27.05.2003
(51) Int. Cl.: A62B 18/08, A62B 23/02, A41D 13/11

(54) **Health mask**

(30) Priority: 28.05.2002 KR 2002016221
(71) Applicant: Bookyung Lee, Ahnyang-City, Kyunggi-Do (KR)
(72) Inventor: Bookyung Lee, Ahnyang-City, Kyunggi-Do (KR)
(74) Representative: Jenkins, Peter David

(57) **Abstract**

In this invention, air-blocking material is contained that enables the formation of a concentrated passage in the inner surface of the mask body that covers the nose and mouth of the user where air is concentrated and passed through at the time of inhalation and exhalation. Accordingly, concentrated passage maintains humidity and preserves heat due to the humidity and warm air of the respiration heat, produced at the time of exhalation. Consequently, cold and dry air of the outside that is the main cause of cold outbreak is converted into warm and humid air while passing through the concentrated passage, which in turn prevents worsening of cold symptoms and the outbreak of cold.

Moreover, frosting of surface of eyeglasses is prevented by ensuring that the hot steam of breath or nose does not flow out towards the upper part of the Mask when the patient who wears eyeglasses wears eyeglasses. As a consequence, user of eyeglasses can wear the Mask, free of inconvenience, and the air-blocking material, comprised of light, soft and elastic material maintains the hanging string and both sides of the ears elastic. Accordingly, pain that might otherwise result from the pressing down of hanging string is prevented, which increases the comfortable feel of wearing the Mask.

## Description

The present invention relates to a health mask, and more particularly to, a health mask that contains air-blocking material that blocks and curtails the passage whereby cold air is inhaled in, and that contains supporting cloth that preserves respiration heat and humidity, produced and discharged at the time of breathing and that curtails the influx of cold air, based of the single layer, into the respiratory organ, thus maximizes inhalation of humid air into the respiratory organ at the time when the user inhales, minimizes inhalation of dry and cold air from the outside, which exerts negative influence on the cold symptoms. The present invention pertains to health mask that underwent improvement to prevent production of condensation and/or frost on the eyeglasses of the users at the time of exhalation.

Generally, health mask (hereafter, "Mask") is used by a patient who is afflicted with illness related to respiratory organ such as coughing or cold to avoid direct contact with the cold and dry air of outside at the time of inhalation.

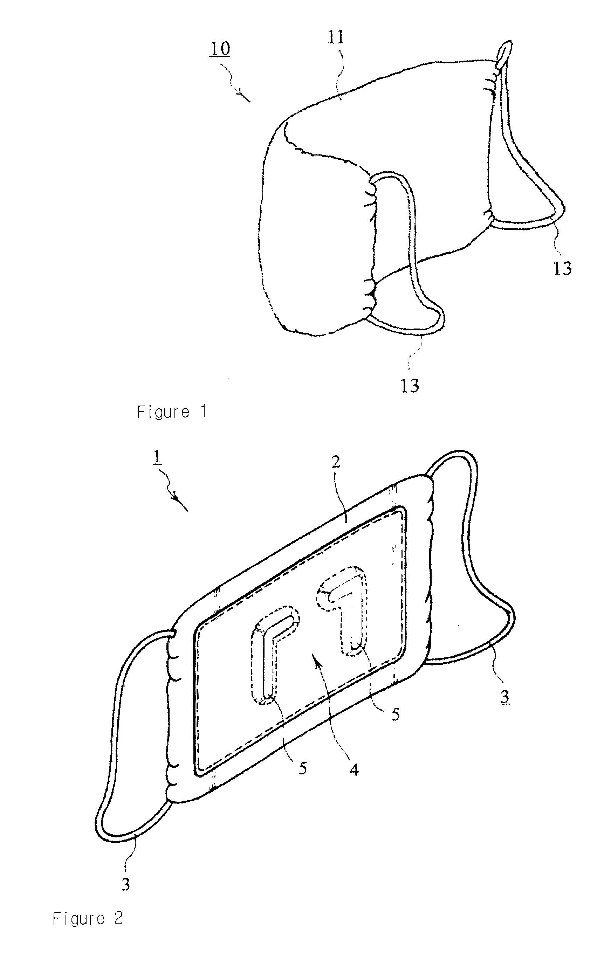

As illustrated by Figure 1, this Mask (10) is comprised of rectangular body (11), which is made of cloth material and which assumes a size that can simultaneously cover user's nose and mouth, and of hanging string (13), attached on both left and right of the aforementioned body (11) to be hanged on user's ears. Symbol on Figure 1 that is not explained is the needlework line that ensures the overlapping of the outer and inner cloth.

On the other hand, patient with cold may mitigate cold symptoms only when humid air is continuously inhaled, and not cold and dry air. When a patient with cold applies and uses general mask (10), the function of a mask is not optimized since inhalation of cold and dry air from the outside by the user takes place through influx passage, which is a space of specific size and which forms between the left and right side of the user's face and the nose ridge that faces and comes into contact with the inner surface of the aforementioned body (11) at the time of inhalation.

In case of user who wears eyeglasses, the user is inconvenienced with unclear view due to the creation of condensation and/or frost on the surface of the eyeglasses due to the hot breathing air, discharged into the empty space situated between the nose ridge and left and right side of the face at the time of exhalation.

Moreover, although it is advisable for the aforementioned body (11) to assume relative humidity from the steam of breath, discharged to the outside and from the nose discharged air so that transformation into humid air may take place as the dry air from the outside, inhaled from the outside at the time of inhalation by the user, passes through the cloth material that comprises the aforementioned body (11), there is limit to maintaining the humidity of the aforementioned body (11) at a consistent level since respiration heat such as steam of nose and steam of breath, incurred at the time of exhalation by the user, is distributed to the entire aforementioned body (11) and then discharged to the outside.

The present invention pertains to health mask. When it pertains to a health mask that is comprised of body (2), large enough to cover nose and mouth since made of cloth material, hanging string (3) that hangs from the ears since installed at the right and left side of aforementioned body (2), supporting cloth (4) attached onto the inner surface of the body (2) that corresponds to the aforementioned nose and mouth, and the air-blocking material (5) that is fixed after symmetrical arrangement into a pair of left and right between the inner and outer cloth (4a) (4b) of the aforementioned supporting cloth (4) to form concentrated passage that fills up the empty space found between the left and right side of the nose and where the steam of breath and nose is concentrated at the inner surface of the body (2). Contains air-blocking material that blocks and curtails influx of cold air. Curtails influx of cold air into the respiratory organ due to the single layer. Contains supporting cloth that preserves respiration heat and humidity that are discharged after being produced at the time of breathing. Maximizes inhalation of humid air into the respiratory organ at the time when the user inhales, minimizing inhalation of dry air from the outside, which aggravates cold symptoms. Prevents creation of frost on the eyeglasses at the time of exhalation.

The aforementioned aspects and other features of the present invention will be explained in the following description, taken in conjunction with the accompanying drawings, wherein:
Figure 1: illustrates general health mask,
Figure 2: illustrates outside features of the health mask, developed according to the present invention,
Figure 3: illustrates health mask, developed according to the present invention,
Figure 4: illustrates usage state of health mask according to the present invention.

### Representative Figure

Figure 2
Indices
mask, cold, supporting cloth, inner and outer cloth, air-blocking material

| * Explanation of symbols found in the key parts of figure * | | | |
|---|---|---|---|
| 1 | mask | 2 | body |
| 3 | hanging string | 4 | supporting cloth |
| 4a, 4b | inner and outer cloth | 5 | air-blocking material |

The present invention will be described in detail by way of a preferred embodiment with reference to accompanying drawings.

Thus, the present invention is recommended as a measure to eradicate hitherto and aforementioned problems. The purpose of the present invention is to offer a health mask that maintains the level of humidity in a way that mask body is not dried off by concentrating the passage where steam of breath and nose is discharged from at the time of exhalation, and which enables user to inhale humid air by converting the air into humid air at the time when cold and dry air from outside passes through the body of the mask at the time of inhalation.

### Composition and Reaction of the Invention

As technological composition with the end of achieving aforementioned purpose, the present invention is comprised of cloth material, body with appropriate size to cover the nose and mouth and with string that is installed on the left and right side of the aforementioned body and which hangs from both ears., and of supporting cloth that is attached onto the inner surface of the body that corresponds to the aforementioned nose and mouth.

The present invention fills up the empty space, formed between the inner surface of the aforementioned body and both left and right side of the nose, and the concentrated passage with concentration of steam of breath and nose is symmetrical arrangement into single pair of left and right between the inner and outer cloth of the aforementioned supporting cloth so that body inner surface that coincide between aforementioned nose and mouth is formed. The present health mark is characterized by the inclusion of air-blocking material subject to fixation.

The following is a more detailed description of the present invention

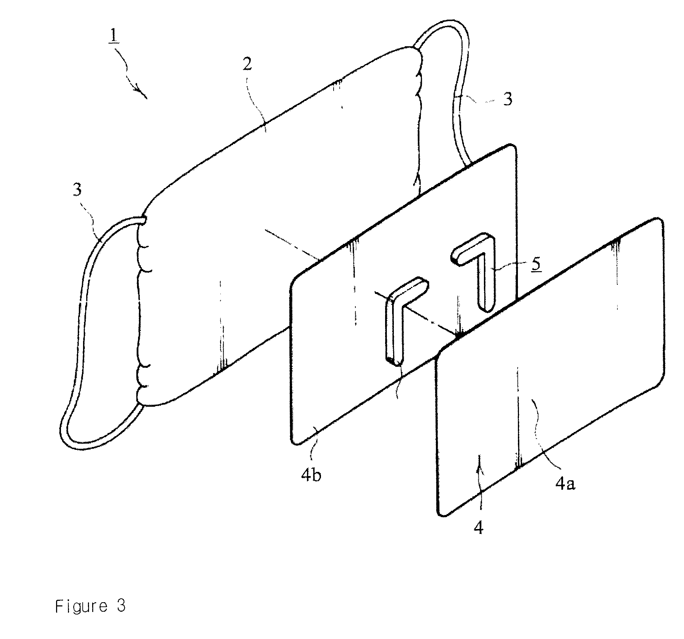

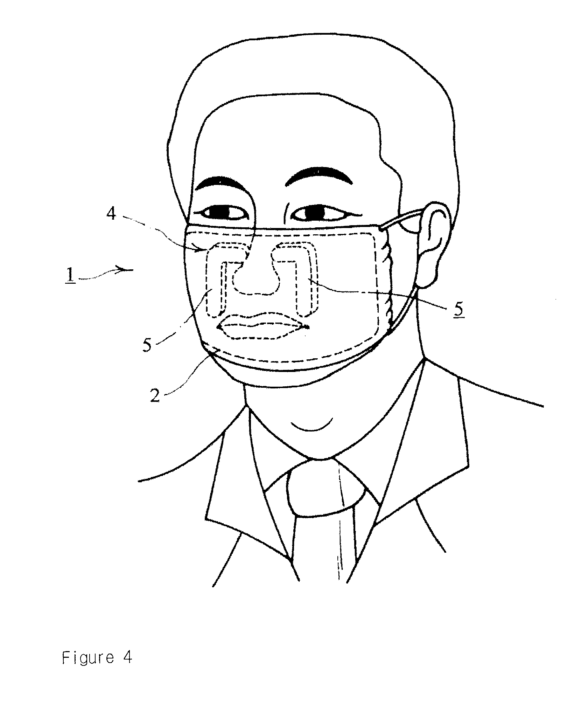

Figure 2 is the external figure of the health mask as resulting from the present invention, Figure 3 is the figure on decomposition that figures the health mask in accordance with the present invention, and Figure 4 is the status of use of the health mark in accordance to the present invention.

As figured on Figure 2 or 4, the Mask (1) of the present invention is sanitary consumable that covers part of the face to minimize the direct contact of the patient with cold from the cold and dry air of the exterior. The present mask (1) is comprised of body (2), hanging string (3), supporting cloth (4) and air-blocking material (5).

In other words, aforementioned body (2) is comprised of rectangular type of cloth material which assumes a size sufficient enough to cover nose and mouth where respiration heat such as stem of breath and nose is discharged to the outside and where outside air is inhaled from the outside at the time of inhalation and exhalation. On the left and right sides of the aforementioned body (2), hanging string (3) is attached to enable user to easily hang the string on both ears at the time of applying the Mask.

And, aforementioned supporting cloth (4) is attached on to the entire inner surface of the body (2) that mutually corresponds to the nose and mouth that is subject to covering, and it is the cloth material that is sewn by overlapping inner and outer cloth (4a) (4b). Here, aforementioned supporting cloth (4) is advised to either be composed of same cloth material as the aforementioned body (2) or of cloth material with outstanding ability to preserve humidity and temperate while facilitating the passage of steam of breath and nose into the interior and exterior.

Furthermore, aforementioned supporting cloth (4) retains air-blocking material (5) of the "¬ "-shape, situated on the device intended for blocking the air and which mutually symmetrical arrangement in the left and right pair, centered on the aforementioned nose, and the aforementioned air-blocking material (5) fills up the empty space that forms between the inner surface of the aforementioned body (2) and both left and right side of the nose, and it is installed in a fixed manner between the inner and outer cloth (4a) (4b) of the aforementioned supporting cloth (4) where the steam of breath and nose is concentrated so that a concentrated passage steam can be formed. At the above mentioned time, it is advised that the aforementioned air-blocking material (5) is sewn onto the supporting cloth (4) so that it does not move among inner and outer cloth (4a) (4b).

The upper part of the air-blocking material (5) curves horizontally so that it comes into contact with the left and right side of the nose. The lower part is advised to assume "¬"-shaped device for blocking the air that expands vertically so that it will near the left and right sides of the lip. Use of flexible, elastic, light and soft material such as sponge is advised for use in the aforementioned air-blocking material (5).

Moreover, supporting cloth (4) that has the aforementioned air-blocking material (5) is advised to use Velcro tape or other adhesive to get composed into the aforementioned body (2) on the left and right sides of the frontal part so that it can be attached easily into the inner surface of the aforementioned body (2) in the form of impact.

And, it is advised that the concentrated passage, formed on the inner surface of the aforementioned body (2), is situated at the inner surface of the body, that corresponds to the space between the aforementioned nose and mouth, so that the part where steam of breath and nose passes through at the time of exhalation and the part where the air is inhaled from the exterior at the time of inhalation coincide as one.

The following is detailed explanation on the reaction and effect of the present innovation, which assumes the above mentioned composition.

When a patient with cold applies and uses the Mask (1) of the present invention, supporting cloth (4), comprised of inner and outer cloth (4a) (4b), is installed at the part that corresponds with the nose and mouth of the patient with cold onto the inner surface of the body (2) that comprises the aforementioned mask (1). Between the aforementioned inner and outer cloth (4a) (4b), the empty space that forms between the inner surface of the aforementioned body (2) and the left and right side of the nose is filled. air-blocking material (5) of left and right pair is installed to form concentrated passage where steam of breath and nose is concentrated.

Given the above mentioned condition, respiration heat such as steam of breath and nose that stems out of the mouth and nose at the time of exhalation by the user is not leaked out to the upper part or to the sides due to the aforementioned air-blocking material (5). Instead, the above mentioned is discharged to the exterior through the body's concentrated passage that pertains to the area between the aforementioned nose and mouth. At the above mentioned time, cloth material that pertains to the concentrated passage of the aforementioned body (2) assumes a higher temperature compared to the atmospheric temperature due to the humidity and warm air, included in the steam of breath and nose that is discharged to the exterior by going through the aforementioned body (2) and maintains the state of humidity and heat preservation due to humidity while user applies on the Mask.

In succession, air from the outside is inhaled into the body through the nose and mouth after going through a concentrated passage at the time of inhalation. At the above mentioned time, inhalation of the air from the outside takes place through the concentrated passage that maintains state of heat and humidity preservation due to repetitive exhalation. Accordingly, the above mentioned becomes a major cause of cold, and cold and dry air that can exert negative influence on the patient with cold at the time of direct contact is transformed into warm and humid air by enabling inhalation into respiratory organ through user's mouth and nose. As a consequence, the present invention prevents worsening of symptoms for the patient with cold and can improve related illness.

Moreover, when patient who wears eyeglasses applies the Mask (1) during winter, creation of frost on the surface of eyeglasses due to the steam of breath or nose is prevented since warm steam of breath or nose is not discharged to the upper part due to the air-blocking material (5) that is placed at the inner surface of the body (2), and since most of the discharged steam goes through via concentrated passage. Accordingly, user of eyeglasses can apply the Mast with utmost convenience.

Moreover, air-blocking material (5) that forms concentrated passage since arranged between the aforementioned body (2) and on the entire surface of the face is comprised of soft, light and elastic material. Accordingly, elasticity is induced at the time of wearing the Mask, which in turn induces elasticity in the area located between two ears and in the hanging string (3), attached onto the left and right side of the aforementioned body (2). As a consequence, pain in both ears, caused by the pressing down of the aforementioned hanging string (3) maybe prevented, increasing the comfort of wearing the Mask.

The present invention provides explanation and figures pertaining to specific examples. It is noteworthy to mentioned that anyone in the applicable industry with expected level of pertinent knowledge understand that the present invention maybe reformed and changed in diverse forms within the boundaries pertaining to the spirit and pertinent area of the present invention mentioned hereafter, in the section of the scope of claim.

## Claims

1. A health mask comprising a substrate for covering the nose and mouth of a user, attachment means on the substrate for attaching the health mask to the head of a user, an inner material layer disposed on an inner surface of the substrate, and an air blocker disposed adjacent to the inner material layer, the air blocker being shaped to restrict upward movement in the health mask of air exhaled from the nose and/or mouth of the user.

2. A health mask according to claim 1 wherein the air blocker comprises two bodies symmetrically arranged on opposed lateral sides of the health mask, the bodies defining a central upper passage for receiving the nose of a user.

3. A health mask according to claim 2 wherein each body is L-shaped, and includes an upper horizontal part and a lower vertical part depending therefrom, the opposed horizontal parts pointing towards each other to define the central upper passage therebetween, and the lower parts being horizontally spaced from each other.

4. A health mask according to any foregoing claim wherein the air blocker is provided between the substrate and the inner layer

5. A health mask according to claim 4 further comprising an intermediate material layer which is located between the substrate and the inner layer, and wherein the air blocker is provided between the inner layer and an intermediate material layer.

6. A health mask, comprised of a body (2) that is large enough to cover the nose and mouth since comprised of cloth material, and hang string (3) that hangs onto the ears which is installed at the left and right side of the body, the health mask filling up the empty space formed between the left and right side of the nose and the inner surface of the body (2) and which is attached onto a supporting cloth (4) that corresponds to the nose and mouth, **characterized by** the inclusion of air-blocking material (5) after being fixed in the state of symmetrical arrangement into the left and right pair between the inner and outer cloth (4a) (4b) to ensure formation of concentrated passage into the inner surface of the body (2) that corresponds to the area between the nose and mouth and where the steam of breath and nose is concentrated.

7. A health mask according to Claim 6, wherein the upper part of the air-blocking material (5) is curved horizontally so that the upper part comes into contact with the left and right side of the nose, and the lower part is formed in the shape of "¬ " in the single surface level, which extends vertically, so that the lower part is the left and right side of the lips.

8. A health mask according to Claim 6, wherein supporting cloth (4) that retains aforementioned air-blocking material (5) is installed on the aforementioned body (2) in a way that the former can be attached onto the inner surface of the aforementioned body (2) in an easy manner in the form of impact, foe example by attachment material such as Velcro tape which is used on the left and right side of the entire surface.
